(19) Europäisches Patentamt
European Patent Office
Office européen des brevets

(11) Veröffentlichungsnummer: **0 310 732 B1**

(12) **EUROPÄISCHE PATENTSCHRIFT**

(45) Veröffentlichungstag der Patentschrift: **11.03.92**

(51) Int. Cl.$^5$: **C07C 231/00**, C07C 235/00, C07C 233/13, C07D 307/33

(21) Anmeldenummer: **87810582.4**

(22) Anmeldetag: **09.10.87**

# (54) Verfahren zur Herstellung von N-Acyl-N-alkyl-2,6-dialkyl-3-chloranilinen.

(43) Veröffentlichungstag der Anmeldung:
**12.04.89 Patentblatt 89/15**

(45) Bekanntmachung des Hinweises auf die Patenterteilung:
**11.03.92 Patentblatt 92/11**

(84) Benannte Vertragsstaaten:
**AT BE CH DE ES FR GB GR IT LI NL**

(56) Entgegenhaltungen:
**GB-A- 1 577 702**
**GB-A- 2 037 746**
**GB-A- 2 098 210**

(73) Patentinhaber: **CIBA-GEIGY AG**
**Klybeckstrasse 141**
**CH-4002 Basel(CH)**

(72) Erfinder: **Eckhardt, Wolfgang, Dr.**
**Breslauerstrasse 14**
**W-7850 Lörrach(DE)**
Erfinder: **Süess, Hans, Dr.**
**Burgunderweg 5**
**CH-4313 Möhlin(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegende Erfindung betrifft ein Verfahren zur Herstellung von N-Acyl-N-alkyl-2,6-dialkyl-3-chloranilinen der Formel I

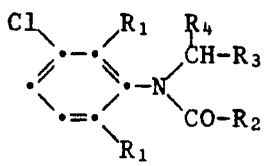

(I)

in welcher $R_1$ Methyl oder Aethyl bedeutet, $R_2$ Alkoxymethyl, Chlormethyl oder 2-Tetrahydrofuryl darstellt, $R_3$ für Alkoxymethyl, Carboxy oder Alkoxycarbonyl steht und $R_4$ Wasserstoff, Methyl und, wenn $R_3$ Carboxy oder Alkoxycarbonyl bedeutet, auch 2-Alkoxyäthyl und 2-Alkoxypropyl darstellt, wobei $R_3$ und $R_4$ zusammen mit dem Kohlenstoffatom, an das beide Reste gebunden sind, auch einen 2-Oxotetrahydro-3-furyl- oder einen 2-Oxo-5-methyltetrahydro-3-furylrest bilden können.

Die Verbindungen der Formel I besitzen zum Teil fungizide und zum Teil herbizide Wirkung. Verbindungen dieser Art sind beispielsweise in den US-Patentschriften 4 564 629 und 3 933 860, der Britischen Patentschrift 1 455 471, der publizierten Europäischen Patentanmeldung EP-A-0 028 011 und der publizierten Britischen Patentanmeldung GB-A-2 006 783 beschrieben.

Aus der publizierten Britischen Patentanmeldung GB-A-2 098 210 ist es bekannt, N-acylierte N-Alkoxycarbonylmethyl- und N-(1-Alkoxycarbonyläthyl)-2,6-dialkyl-3-halogenaniline durch Einwirkung von Halogen auf entsprechende N-Alkoxycarbonylmethyl- und N-(1-Alkoxycarbonyläthyl)-2,6-dialkylaniline in Gegenwart von mindestens 2 Mol Lewis-Säure pro Mol N-Alkoxycarbonylalkyl-2,6-dialkylanilin und nachfolgende Acylierung der erhaltenen N-Alkoxycarbonylmethyl und N-(1-Alkoxycarbonyläthyl)-2,6-dialkyl-3-halogenaniline herzustellen. Dabei kommen als Lewis-Säuren Aluminiumchlorid, Aluminiumbromid, Bortrifluorid, Zinntetrachlorid und Titantetrachlorid in Betracht. Dieses Verfahren ist insofern nachteilig, als zur Erreichung des gewünschten Effekts sehr grosse Mengen an Lewis-Säure eingesetzt werden müssen. Beispielsweise sind bei Verwendung von Aluminiumchlorid pro Gewichtsteil N-Alkoxycarbonylalkyl-2,6-dialkylanilin mindestens 2 Gewichtsteile Aluminiumchlorid erforderlich. Da diese grosse Menge Aluminiumchlorid vor der Aufarbeitung des Reaktionsgemisches zunächst mit Wasser zersetzt werden muss, ist das Verfahren sowohl in bezug auf die benötigten Ausgangsmaterialien und Hilfsstoffe als auch in bezug auf seine Durchführung aufwendig.

Es ist ferner bekannt, 3-Chlor-2,6-dimethylacetanilid durch Chlorierung von 2,6-Dimethylacetanilid in einer Ausbeute von 80 % der Theorie herzustellen (vgl. Synthesis, 1971, Seite 467). Unter Verwendung dieser Methode sind die N-Acyl-N-alkyl-2,6-dialkyl-3-chloraniline der Formel I ausgehend von einem entsprechenden 2,6-Dialkylanilin durch Acetylierung, Chlorierung des 2,6-Dialkylacetanilids, Hydrolyse des gebildeten 3-Chlor-2,6-Dialkylacetanilid zum 3-Chlor-2,6-dialkylanilin, dessen Alkylierung und anschliessende Acylierung nach folgendem Schema zugänglich:

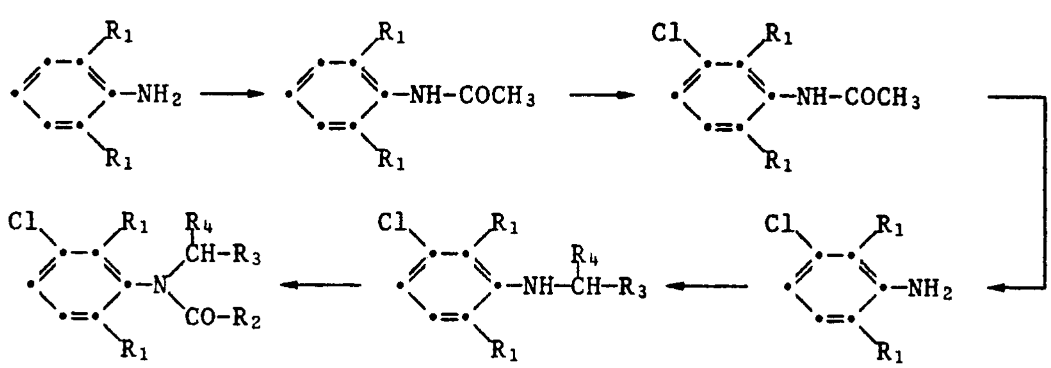

Nach dieser Methode können die N-Acyl-N-alkyl-2,6-dialkyl-3-chloraniline der Formel I bezogen auf das als Ausgangsmaterial verwendete 2,6-Dialkyldianilin in einer Ausbeute von etwa 40 % der Theorie hergestellt werden. Die Methode ist wegen der grossen Zahl der benötigten Reaktionsstufen umständlich und

vermag hinsichtlich der erzielbaren Ausbeuten nicht zu befriedigen.

Es ist daher das Ziel der vorliegenden Erfindung, ein Verfahren bereitzustellen, das die Herstellung der N-Acyl-N-alkyl-2,6-dialkyl-3-chloraniline der Formel I in einfacher Weise und in guter Ausbeute ermöglicht.

Es wurde gefunden, dass dieses Ziel in vorteilhafter Weise erreicht werden kann, wenn man von einem entsprechenden 2,6-Dialkylanilin ausgeht, dieses durch Alkylierung und nachfolgende Acylierung in ein entsprechendes N-Acyl-N-alkyl-2,6-dialkylanilin überführt und dieses dann durch Einwirkung von Chlor in ein N-Acyl-N-alkyl-2,6-dialkyl-3-chloranilin der Formel I umsetzt.

Gemäss vorliegender Erfindung wird daher vorgeschlagen, die N-Acyl-N-alkyl-2,6-dialkyl-3-chloraniline der Formel I in der Weise herzustellen, dass man ein 2,6-Dialkylanilin der Formel II

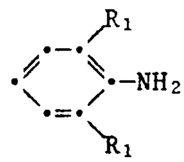

(II)

in welcher $R_1$ die unter Formel I angegebene Bedeutung hat, mit einem Halogenid der Formel III

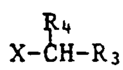

(III)

in welcher $R_3$ und $R_4$ die unter Formel I angegebene Bedeutung haben und X für Chlor oder Brom steht, zu einem N-Alkyl-2,6-dialkylanilin der Formel IV

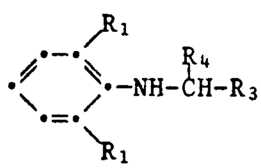

(IV)

in welcher $R_1$, $R_3$ und $R_4$ die unter Formel I angegebene Bedeutung haben, umsetzt, das N-Alkyl-2,6-dialkylanilin der Formel IV anschliessend mit einem Acylierungsmittel der Formel V

$X_1$-CO-$R_2$ (V)

in welcher $R^2$ die unter Formel I angegebene Bedeutung hat und $X_1$ Chlor, Brom oder -O-CO-$R_2$ bedeutet, umsetzt und das erhaltene N-Acyl-N-alkyl-2,6-dialkylanilin der Formel VI

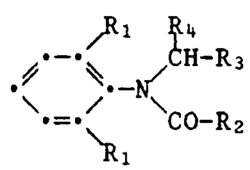

(VI)

in welcher $R_1$, $R_2$, $R_3$ und $R_4$ die unter Formel I angegebene Bedeutung haben, anschliessend durch Einwirkung von Chlor in ein N-Acyl-N-alkyl-2,6-dialkyl-3-chloranilin der Formel I überführt.

Geeignete 2,6-Dialkylaniline der Formel II sind 2,6-Dimethylanilin und 2,6-Diäthylanilin. Besonders geeignet ist 2,6-Dimethylanilin.

Geeignete Halogenide der Formel III sind 2-Alkoxyäthylchlorid, 2-Alkoxyäthylbromid, 2-Alkoxypropylchlorid, 2-Alkoxypropylbromid, 2-Chloressigsäure, 2-Bromessigsäure, 2-Chlorpropionsäure, 2-Brompropionsäure, 2-Chloressigsäurealkylester, 2-Bromessigsäurealkylester, 2-Chlorpropionsäurealkylester, 2-Brompropionsäurealkylester, 2-Chlor-4-alkoxybuttersäure, 2-Brom-4-alkoxybuttersäure, 2-Chlor-4-alkoxyvaleriansäure, 2-Brom-4-alkoxyvaleriansäure, 2-Chlor-4-alkoxybuttersäurealkylester, 2-Brom-4-alkoxybuttersäurealkylester, 2-Chlor-4-alkoxyvaleriansäurealkylester, 2-Brom-4-alkoxy-valeriansäurealkylester, α-Chlor-γ-butyrolacton, α-Brom-γ-butyrolacton, α-Chlor-γ-valerolacton und α-Brom-γ-valerolacton, wobei die in den vorgenannten Halogeniden der Formel III vorkommenden Alkoxy- und Alkylestergruppen Alkylreste mit jeweils 1-4 Kohlenstoffatomen enthalten. Diese Alkylreste können im einzelnen Methyl, Aethyl, Propyl, Isopropyl, Butyl, sec. Butyl, tert. Butyl und Isobutyl darstellen.

Bei Verwendung von Halogeniden der Formel III, in welcher X Chlor bedeutet, wird die Umsetzung eines 2,6-Dialkylanilins der Formel II mit dem Halogenid der Formel III vorteilhaft in Gegenwart eines Alkalijodids, insbesondere Kaliumjodid, als Katalysator durchgeführt.

Bevorzugte Halogenide der Formel III sind 2-Methoxyäthylchlorid, 2-Aethoxyäthylchlorid, 2-Methoxy-1-methyläthylchlorid, 2-Chloressigsäuremethylester, 2-Chloressigsäureäthylester, 2-Brompropionsäuremethylester, 2-Brompropionsäureäthylester und α-Chlor-γ-butyrolacton. Ein besonders bevorzugtes Halogenid der Formel III ist α-Brom-γ-butyrolacton.

Geeignete Acylierungsmittel der Formel V sind Chloride und Bromide der Chloressigsäure, Alkoxyessigsäure und Tetrahydrofuran-2-carbonsäure, sowie die Anhydride dieser Säuren, wobei unter Alkoxyessigsäuren insbesondere solche mit einem $C_1$-$C_4$-Alkoxyrest, wie Methoxy, Aethoxy, Propoxy, Isopropoxy, Butoxy, sek. Butoxy, tert. Butoxy, und Isobutoxy, zu verstehen sind. Bevorzugte Acylierungsmittel sind Methoxyacetylchlorid, Chloracetylchlorid und Tetrahydrofuran-2-carbonsäurechlorid. Besonders bevorzugt ist Methoxyacetylchlorid.

Die Umsetzung eines 2,6-Dialkylanilins der Formel II mit einem Halogenid der Formel III wird vorteilhaft in einem inerten Lösungsmittel in Gegenwart eines säurebindenden Mittels durchgeführt. Als inerte Lösungsmittel kommen aromatische Kohlenwasserstoffe und Halogenkohlenwasserstoffe, wie Benzol, Toluol, Xylol, Chlorbenzol und o-Dichlorbenzol, sowie N,N-disubstituierte Carbonsäureamide, wie N,N-Dimethylformamid und N,N-Dimethylacetamid, sowie überschüssiges 2,6-Dialkylanilin der Formel II in Betracht. Bevorzugte Lösungsmittel sind Toluol und Xylol. Als säurebindende Mittel können anorganische und organische Basen, wie Alkali- und Erdalkalimetallhydroxide, -carbonate und -hydrogencarbonate, Triäthylamin, Pyridin oder überschüssiges 2,6-Dialkylanilin der Formel II verwendet werden. Eine bevorzugte Base ist Natriumcarbonat. Die Reaktionstemperaturen liegen in der Regel zwischen 80°C und Rückflusstemperatur des Reaktionsmediums. Vorteilhaft wird die Alkylierung bei Rückflusstemperatur des Reaktionsmediums durchgeführt. Die Umsetzung eines 2,6-Dialkylanilins der Formel II mit einem Halogenid der Formel III wird daher vorzugsweise in Toluol oder Xylol als Lösungsmittel in Gegenwart von Natriumcarbonat als säurebindendes Mittel bei Rückflusstemperatur des Reaktionsmediums vorgenommen.

Die Umsetzung eines N-Alkyl-2,6-dialkylanilins der Formel IV mit einem Acylierungsmittel der Formel V wird vorteilhaft in einem inerten Lösungsmittel in Anwesenheit oder Abwesenheit eines säurebindenden Mittels durchgeführt. Als inerte Lösungsmittel kommen insbesondere mit Wasser nicht mischbare Lösungsmittel, wie aliphatische und aromatische Kohlenwasserstoffe und Halogenkohlenwasserstoffe in Betracht. Geeignete Lösungsmittel sind beispielsweise Hexan, Benzol, Toluol, Xylol, Chlorbenzol, Methylenchlorid, Chloroform, Kohlenstofftetrachlorid und Aethylenchlorid. Bevorzugte Lösungsmittel sind Toluol und Xylol.

Als säurebindende Mittel, in deren Gegenwart die Umsetzung eines N-Alkyl-2,6-dialkylanilins der Formel IV mit einem Acylierungsmittel der Formel V durchgeführt werden kann, kommen anorganische und organische Basen, wie Alkali- und Erdalkalimetallhydroxide, -carbonate und -hydrogencarbonate, Triäthylamin und Pyridin in Betracht. Vorzugsweise wird die Umsetzung eines N-alkyl-2,6-dialkylanilins der Formel IV mit einem Acylierungsmittel der Formel V in Abwesenheit einer Base in Toluol oder Xylol und bei vermindertem Druck durchgeführt. Geeignete Drucke, bei denen die Umsetzung eines N-Alkyl-2,6-dialkylanilins der Formel IV mit einem Acylierungsmittel der Formel V durchgeführt werden kann, liegen zwischen 50 und 150 mbar, insbesondere bei 60-100 mbar.

Die Chlorierung eines N-Acyl-N-alkyl-2,6-dialkylanilins der Formel VI wird ebenfalls vorteilhaft in einem inerten Lösungsmittel vorgenommen. Als Lösungsmittel sind insbesondere niedere aliphatische Carbonsäuren, wie Ameisensäure und Essigsäure geeignet. Ferner können chlorierte aromatische und aliphatische Kohlenwasserstoffe, wie Chlorbenzol, Methylenchlorid, Chloroform, Kohlenstofftetrachlorid und Aethylenchlorid verwendet werden. Die als Lösungsmittel verwendbaren Carbonsäuren können bis zu 60 Gew.-% Wasser enthalten. Ein bevorzugtes Lösungsmittel, in dem die Chlorierung eines N-Acyl-N-alkyl-2,6-dialkylanilins der Formel VI durchgeführt werden kann, ist Ameisensäure mit einem Wassergehalt von bis zu 40 Gew.-%.

Die Chlorierung wird vorteilhaft bei Temperaturen von 20-40°C durchgeführt. Die Chlorierung kann auch bei höheren oder tieferen Temperaturen durchgeführt werden. Es ist jedoch zu beachten, dass bei Temperaturen über 40°C zunehmend dichlorierte Produkte gebildet werden, während bei Temperaturen unter 20°C die Gefahr besteht, dass die Reaktion nicht mehr sofort beim Beginn des Einleitens von Chlor einsetzt, sondern erst in Gang kommt, wenn sich bereits eine relativ hohe Konzentration an Chlor aufgebaut

hat. Diese verspätet einsetzende Reaktion verläuft dann sehr heftig und es ist schwierig, die Temperatur des Reaktionsgemisches zu kontrollieren. Auch in diesem Fall muss mit der Bildung von dichlorierten Produkten gerechnet werden.

Es ist ferner vorteilhaft, die Chlorierung in Gegenwart von Lewis-Säuren, wie Aluminiumchlorid, Eisen(III)chlorid, Bortrifluorid, und Titantetrachlorid durchzuführen. Eine bevorzugte Lewis-Säure ist Eisen(III)-chlorid. Die Lewis-Säuren werden in einer Menge von 1-5 Gew.%, vorzugsweise 1,5-2,5 Gew.% bezogen auf das zu chlorierende N-Acyl-N-alkyl-2,6-dialkylanilin der Formel VI eingesetzt. Die Lewis-Säuren habe an sich keinen wesentlichen Einfluss auf die Chlorierung, sie bewirken jedoch eine starke Erhöhung der Löslichkeit der zu chlorierenden N-Acyl-N-alkyl-2,6-dialkylaniline der Formel VI in wässriger Ameisensäure oder wässriger Essigsäure. Daher empfiehlt sich der Zusatz von Lewis-Säuren insbesondere bei Verwendung von Ameisensäure oder Essigsäure als Lösungsmittel, um eine höhere Volumenausbeute zu erreichen. Die Chlorierung eines N-Acyl-N-alkyl-2,6-dialkylanilins der Formel VI wird daher vorzugsweise bei 20-40 °C in Ameisensäure mit einem Wassergehalt von bis zu 40 Gew.% in Gegenwart von 1,5-2,5 Gew.% Eisen(III)chlorid bezogen auf eingesetztes N-Acyl-N-alkyl-2,6-dialkylanilin der Formel VI durchgeführt.

Die Chlorierung wird in der Regel bei Normaldruck durchgeführt. Bei Verwendung von Ameisensäure oder Essigsäure als Lösungsmittel kann auch unter leichtem Ueberdruck gearbeitet werden, da bei Verwendung dieser Lösungsmittel kein Gas aus dem Reaktionsgemisch entweicht.

Durch das erfindungsgemässe Verfahren wird es möglich, die N-Acyl-N-alkyl-2,6-dialkyl-3-chloraniline der Formel I ausgehend von 2,6-Dialkylanilinen der Formel II in einfacher Weise und in wesentlich besserer Ausbeute herzustellen als mit den bekannten Verfahren. Gegenüber dem in der GB-A-2 089 210 beschriebenen Verfahren, das auf der Chlorierung von N-Alkyl-2,6-dialkylanilinen in Gegenwart von mindestens 2 Mol einer Lewis-Säure beruht, entfällt die Verwendung grosser Mengen von Lewis-Säure und die damit verbundenen Schwierigkeiten bei der Aufarbeitung. Gegenüber dem eingangs genannten Verfahren, das auf der Chlorierung von N-Acetyl-2,6-dialkylanilinen beruht, entfallen zwei Reaktionsstufen, nähmlich die Einführung der Acetylgruppe vor der Chlorierung und ihre Abspaltung nach der Chlorierung. Ferner verläuft die N-Alkylierung von 2,6-Dialkylanilinen mit besserer Ausbeute als die N-Alkylierung entsprechender 2,6-Dialkyl-3-chloraniline. Ausserdem werden überraschenderweise bei der Chlorierung von N-Acyl-N-alkyl-2,6-dialkylanilinen der Formel VI bessere Ausbeuten erreicht als bei der bekannten Chlorierung von 2,6-Dialkylacetaniliden. Diese Vorteile werden erst durch das erfindungsgemässe Konzept nutzbar, dessen wesentliches Merkmal darin besteht, dass die Einführung des Chloratoms in 3-Stellung des Phenylrestes in der letzten Stufe durchgeführt wird.

Das erfindungsgemässe Verfahren wird durch das folgende Beispiel näher erläutert.

Beispiel 1: Herstellung von N-Methoxyacetyl-N-(2-oxotetrahydro-3-furyl)-3-chlor-2,6-dimethylanilin.

a) In eine Lösung von 121 g (1,0 Mol) 2,6-Dimethylanilin in 500 ml Xylol werden 64 g (0,6 Mol) wasserfreies Natriumcarbonat suspendiert. In diese Suspension werden bei Rückflusstemperatur (etwa 140 °C) während 2 Stunden 206 g (1,25 Mol) $\alpha$-Brom-$\gamma$-butyrolacton (3-Brom-2-oxotetrahydrofuran) eindosiert. Nach beendigter Zugabe des $\alpha$-Brom-$\gamma$-butyrolactons wird das Reaktionsgemisch 4 Stunden bei Rückflusstemperatur nachgerührt. Das bei der Umsetzung entstehende Wasser wird während der Zugabe des $\alpha$-Brom-$\gamma$-butyrolactons und während des Nachrührens ausgekreist. Danach wird das Reaktionsgemisch auf 50 °C abgekühlt, zunächst mit 200 ml Wasser und dann mit 200 ml 5 %iger Salzsäure gewaschen und anschliessend durch Abdestillieren von 50-60 ml Lösungsmittel getrocknet. Das Reaktionsgemisch enthält dann nach gaschromatographischer Bestimmung 174 g (85 % der Theorie) N-(2-Oxotetrahydro-3-furyl)-2,6-dimethylanilin, das beim Abkühlen des Reaktionsgemisches teilweise ausfällt. Die erhaltene Suspension kann direkt in der nächsten Stufe weiterverarbeitet werden. Das Produkt kann jedoch auch durch Abdestillieren des Lösungsmittels und Kristallisation des Rückstandes aus Isopropanol gewonnen werden. Der Schmelzpunkt beträgt 82-84 °C.

b) In eine Suspension von 205 g (1,0 Mol) N-(2-Oxotetrahydro-3-furyl)-2,6-dimethylanilin in 500 ml Xylol werden bei 60-70 °C und einem Druck von 70-80 mbar während 2 Stunden 113 g (1,04 Mol) Methoxyacetylchlorid eindosiert. Gegen Ende der Eindosierung von Methoxyacetylchlorid kommt das Reaktionsgemisch unter Entwicklung von Chlorwasserstoff zum Sieden. Nach beendigter Zugabe des Methoxyacetylchlorids wird das Reaktionsgemisch bei einer Temperatur von 60-65 °C und einem Druck von 70-80 mbar 3 Stunden unter schwachem Rückfluss nachgerührt und der bei der Reaktion entstandene Chlorwasserstoff entfernt. Danach wird etwa die Hälfte des Xylols abdestilliert, auf 20 °C abgekühlt, das abgeschiedene Produkt abfiltriert, mit Xylol gewaschen und getrocknet. Es werden 263 g (95 % der Theorie) N-Methoxyacetyl-N-(2-oxotetrahydro-3-furyl)-2,6-dimethylanilin vom Schmelzpunkt 118-120 °C erhalten.

c) In eine Lösung von 277 g (1,0 Mol) N-Methoxyacetyl-N-(2-oxotetrahydro-3-furyl)-2,6-dimethylanilin und 5 g Eisen(III)chlorid in 300 ml 85 %iger Ameisensäure werden bei 25-30 °C während 2 Stunden 74,6 g (1,05 Mol) Chlor eingeleitet. Es erfolgt eine exotherme Reaktion, bei der praktisch keine Gasentwicklung eintritt. Nach beendigter Zugabe des Chlors wird das Reaktionsgemisch 30 Minuten bei 25 °C nachgerührt, anschliessend die Ameisensäure im Vakuum abdestilliert, der Rückstand in 500 ml Toluol aufgenommen und die toluolische Lösung mit 100 ml Wasser gewaschen. Der nach dem Abdestillieren des Toluols erhaltene ölige Rückstand wird aus Isopropanol/Hexan kristallisiert. Es werden 287 g (92 % der Theorie) N-Methoxyacetyl-N-(2-oxotetrahydro-3-furyl)-3-chlor-2,6-dimethylanilin vom Schmelzpunkt 80-82 °C erhalten.

## Patentansprüche

**1.** Verfahren zur Herstellung von N-Acyl-N-alkyl-2,6-dialkyl-3-chloranilin der Formel I

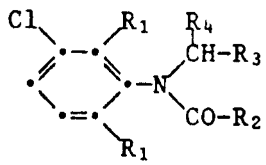

(I)

in welcher $R_1$ Methyl oder Aethyl bedeutet, $R_2$ Alkoxymethyl, Chlormethyl oder 2-Tetrahydrofuryl darstellt, $R_3$ für Alkoxymethyl, Carboxy oder Alkoxycarbonyl steht und $R_4$ Wasserstoff, Methyl und, wenn $R_3$ Carboxy oder Alkoxycarbonyl bedeutet, auch 2-Alkoxyäthyl und 2-Alkoxypropyl darstellt, wobei $R_3$ und $R_4$ zusammen mit dem Kohlenstoffatom, an das beide Reste gebunden sind, auch einen 2-Oxotetrahydro-3-furyl- oder einen 2-Oxo-5-methyltetrahydro-3-furylrest bilden können, dadurch gekennzeichnet, dass man ein 2,6-Dialkylanilin der Formel II

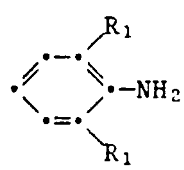

(II)

in welcher $R_1$ die unter Formel I angegebene Bedeutung hat, mit einem Halogenid der Formel III

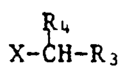

(III)

in welcher $R_3$ und $R_4$ die unter Formel I angegebene Bedeutung haben und X für Chlor oder Brom steht, zu einem N-Alkyl-2,6-dialkylanilin der Formel IV

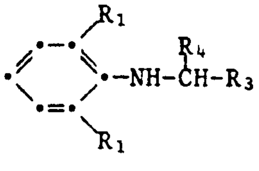

(IV)

in welcher $R_1$, $R_3$ und $R_4$ die unter Formel I angegebene Bedeutung haben, umsetzt, das N-Alkyl-2,6-dialkylanilin der Formel IV anschliessend mit einem Acylierungsmittel der Formel V

$X_1$-CO-$R_2$ (V)

in welcher $R_2$ die unter Formel I angegebene Bedeutung hat und $X_1$ Chlor, Brom oder -O-CO-$R_2$

bedeutet, umsetzt und das erhaltene N-Acyl-N-alkyl-2,6-dialkylanilin der Formel VI

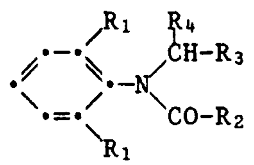

(VI)

in welcher $R_1$, $R_2$, $R_3$ und $R_4$ die unter Formel I angegebene Bedeutung haben, anschliessend durch Einwirkung von Chlor in ein N-Acyl-N-alkyl-2,6-dialkyl-3-chloranilin der Formel I überführt.

**2.** Verfahren nach Anspruch 1, dadurch gekennzeichnet, dass man als 2,6-Dialkylanilin der Formel II 2,6-Dimethylanilin oder 2,6-Diäthylanilin verwendet.

**3.** Verfahren nach Anspruch 2, dadurch gekennzeichnet, dass man als 2,6-Dialkylanilin der Formel II 2,6-Dimethylanilin verwendet.

**4.** Verfahren nach Anspruch 1, dadurch gekennzeichnet, dass man als Halogenid der Formel III 2-Methoxyäthylchlorid, 2-Aethoxyäthylchlorid, 2-Methoxy-1-methyläthylchlorid, 2-Chloressigsäuremethylester, 2-Chloressigsäureäthylester, 2-Brompropionsäuremethylester oder $\alpha$-Brom-$\gamma$-butyrolacton verwendet.

**5.** Verfahren nach Anspruch 4, dadurch gekennzeichnet, dass man als Halogenid der Formel III $\alpha$-Brom-$\gamma$-butyrolacton verwendet.

**6.** Verfahren nach Anspruch 1, dadurch gekennzeichnet, dass man als Acylierungsmittel der Formel V Methoxyacetylchlorid, Chloracetylchlorid oder Tetrahydrofuran-2-carbonsäurechlorid verwendet.

**7.** Verfahren nach Anspruch 6, dadurch gekennzeichnet, dass man als Acylierungsmittel der Formel V Methoxyacetylchlorid verwendet.

**8.** Verfahren nach Anspruch 1, dadurch gekennzeichnet, dass man die Umsetzung eines 2,6-Dialkylanilins der Formel II mit einem Halogenid der Formel III in einem inerten Lösungsmittel in Gegenwart eines säurebindenden Mittels durchführt.

**9.** Verfahren nach Anspruch 8, dadurch gekennzeichnet, dass man als inertes Lösungsmittel Benzol, Toluol, Xylol, Chlorbenzol, o-Dichlorbenzol, N,N-Dimethylformamid oder N,N-Dimethylacetamid verwendet.

**10.** Verfahren nach Anspruch 8, dadurch gekennzeichnet, dass man als inertes Lösungsmittel Toluol oder Xylol verwendet.

**11.** Verfahren nach Anspruch 8, dadurch gekennzeichnet, dass man als säurebindendes Mittel Alkali- und Erdalkalimetalalhydroxide, -carbonate und -hydrogencarbonate, Triäthylamin oder Pyridin verwendet.

**12.** Verfahren nach Anspruch 8, dadurch gekennzeichnet, dass man als säurebindendes Mittel Natriumcarbonat verwendet.

**13.** Verfahren nach Anspruch 1, dadurch gekennzeichnet, dass man die Umsetzung eines 2,6-Dialkylanilins der Formel II mit einem Halogenid der Formel III bei einer Temperatur zwischen 80 ° C und Rückflusstemperatur des Reaktionsmediums durchführt.

**14.** Verfahren nach Anspruch 13, dadurch gekennzeichnet, dass man die Umsetzung eines 2,6-Dialkylanilins der Formel II mit einem Halogenid der Formel III bei Rückflusstemperatur des Reaktionsmediums durchführt.

**15.** Verfahren nach Anspruch 1, dadurch gekennzeichnet, dass man die Umsetzung eines 2,6-Dialkylanilins

der Formel II mit einem Halogenid der Formel III in Toluol oder Xylol als Lösungsmittel in Gegenwart von Natriumcarbonat als säurebindendes Mittel bei Rückflusstemperatur des Reaktionsmediums durchführt.

**16.** Verfahren nach Anspruch 1, dadurch gekennzeichnet, dass man die Umsetzung eines N-Alkyl-2,6-dialkylanilins der Formel IV mit einem Acylierungsmittel der Formel V in einem inerten Lösungsmittel durchführt.

**17.** Verfahren nach Anspruch 16, dadurch gekennzeichnet, dass man die Umsetzung eines N-Alkyl-2,6-dialkylanilins der Formel IV mit einem Acylierungsmittel der Formel V in Hexan, Benzol, Toluol, Xylol, Chlorbenzol, Methylenchlorid, Chloroform, Kohlenstofftetrachlorid oder Aethylenchlorid als Lösungsmittel durchführt.

**18.** Verfahren nach Anspruch 16, dadurch gekennzeichnet, dass man die Umsetzung eines N-Alkyl-2,6-dialkylanilins der Formel IV mit einem Acylierungsmittel der Formel V in Toluol oder Xylol als Lösungsmittel durchführt.

**19.** Verfahren nach Anspruch 1, dadurch gekennzeichnet, dass man die Umsetzung eines N-Alkyl-2,6-dialkylanilins der Formel IV mit einem Acylierungsmittel der Formel V in Abwesenheit einer Base in Toluol oder Xylol als Lösungsmittel bei vermindertem Druck durchführt.

**20.** Verfahren nach Anspruch 1, dadurch gekennzeichnet, dass man die Chlorierung eines N-Acyl-N-alkyl-2,6-dialkylanilins der Formel VI in einem inerten Lösungsmittel durchführt.

**21.** Verfahren nach Anspruch 20, dadurch gekennzeichnet, dass man Ameisensäure, Essigsäure, Chlorbenzol, Methylenchlorid, Chloroform, Kunststofftetrachlorid oder Aethylenchlorid als inertes Lösungsmittel verwendet.

**22.** Verfahren nach Anspruch 20, dadurch gekennzeichnet, dass man Ameisensäure oder Essigsäure mit einem Wassergehalt von bis zu 40 Gew.% als inertes Lösungsmittel verwendet.

**23.** Verfahren nach Anspruch 1, dadurch gekennzeichnet, dass man die Chlorierung eines N-Acyl-N-alkyl-2,6-dialkylanilins der Formel VI bei Temperaturen von 20-40 ° C durchführt.

**24.** Verfahren nach Anspruch 1, dadurch gekennzeichnet, dass man die Chlorierung eines N-Acyl-N-alkyl-2,6-dialkylanilins der Formel VI in Gegenwart von 1-5 Gew.% einer Lewis-Säure durchführt.

**25.** Verfahren nach Anspruch 24, dadurch gekennzeichnet, dass man als Lewis-Säure Aluminiumchlorid, Eisen(III)chlorid, Bortrifluorid oder Titantetrachlorid verwendet.

**26.** Verfahren nach Anspruch 24, dadurch gekennzeichnet, dass man als Lewis-Säure Eisen(III)chlorid verwendet.

**27.** Verfahren nach Anspruch 1, dadurch gekennzeichnet, dass man die Chlorierung eines N-Acyl-N-alkyl-2,6-dialkylanilins der Formel VI bei 20-40 ° C in Ameisensäure mit einem Wassergehalt von bis zu 40 Gew.% in Gegenwart von 1,5-2,5 Gew.% Eisen(III)chlorid bezogen auf eingesetztes N-Acyl-N-alkyl-2,6-dialkylanilin der Formel VI durchführt.

## Claims

**1.** A process for the preparation of an N-acyl-N-alkyl-2,6-dialkyl-3-chloroaniline of formula I

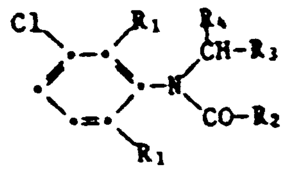

(I)

wherein $R_1$ is methyl or ethyl, $R_2$ is alkoxymethyl, chloromethyl or 2-tetrahydrofuryl, $R_3$ is alkoxymethyl, carboxy or alkoxycarbonyl and $R_4$ is hydrogen or methyl, and, if $R_3$ is carboxy or alkoxycarbonyl, $R_4$ may also be 2-alkoxyethyl or 2-alxoxypropyl, and $R_3$ and $R_4$, together with the carbon atom to which both radicals are attached, may also form a 2-oxotetrahydro-3-furyl radical or a 2-oxo-5-methyltetrahydro-3-furyl radical, which process comprises reacting a 2,6-dialkylaniline of formula II

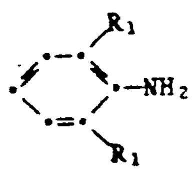

(II)

wherein $R_1$ is as defined for formula I, with a halide of formula III

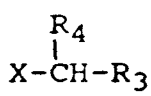

(III)

wherein $R_3$ and $R_4$ are as defined for formula I and X is chlorine or bromine, to give an N-alkyl-2,6-dialkylaniline of formula IV

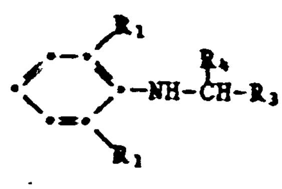

(IV)

wherein $R_1$, $R_3$ and $R_4$ are as defined for formula I, then reacting the N-alkyl-2,6-dialkylaniline of formula IV with an acylating agent of formula V

$X_1$-CO-$R_2$ (V)

wherein $R_2$ is as defined for formula I and $X_1$ is chlorine, bromine or -O-CO-$R_2$, and subsequently converting the resultant N-acyl-N-alxyl-2,6-dialkylaniline of formula VI

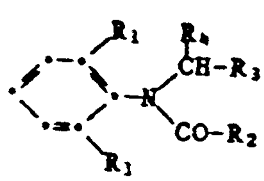

(VI)

wherein $R_1$, $R_2$, $R_3$ and $R_4$ are as defined for formula I, by reaction with chlorine into an N-acyl-N-alkyl-2,6-dialkyl-3-chloroaniline of formula I.

**2.** A process according to claim 1, wherein the 2,6-dialkylaniline of formula II is 2,6-dimethylaniline or 2,6-diethylaniline.

**3.** A process according to claim 2, wherein the 2,6-dialkylaniline of formula II is 2,6-dimethylaniline.

**4.** A process according to claim 1, wherein the halide of formula III is 2-methoxyethyl chloride, 2-ethoxyethyl chloride, 2-methoxy-1-methylethyl chloride, methyl 2-chloroacetate, ethyl 2-chloroacetate, methyl 2-bromopropionate or $\alpha$-bromo-$\gamma$-butyrolactone.

**5.** A process according to claim 4, wherein the halide of formula III is $\alpha$-bromo-$\gamma$-butyrolactone.

**6.** A process according to claim 1, wherein the acylating agent of formula V is methoxyacetyl chloride, chloroacetyl chloride or tetrahydrofuran-2-carboxylic acid chloride.

**7.** A process according to claim 6, wherein the acylating agent of formula V is methoxyacetyl chloride.

**8.** A process according to claim 1, wherein the reaction of a 2,6-dialkylaniline of formula II with a halide of formula III is carried out in an inert solvent and in the presence of an acid acceptor.

**9.** A process according to claim 8, wherein the inert solvent is benzene, toluene, xylene, chlorobenzene, o-dichlorobenzene, N,N-dimethylformamide or N,N-dimethylacetamide.

**10.** A process according to claim 8, wherein the inert solvent is toluene or xylene.

**11.** A process according to claim 8, wherein the acid acceptor is an alkali metal hydroxide, carbonate or bicarbonate, an alkaline earth metal hydroxide, carbonate or bicarbonate, triethylamine or pyridine.

**12.** A process according to claim 8, wherein the acid acceptor is sodium carbonate.

**13.** A process according to claim 1, wherein the reaction of a 2,6-dialkylaniline of formula II with a halide of formula III is carried out at a temperature in the range from 80°C to the reflux temperature of the reaction medium.

**14.** A process according to claim 13, wherein the reaction of a 2,6-dialkylaniline of formula II with a halide of formula III is carried out at the reflux temperature of the reaction medium.

**15.** A process according to claim 1, wherein the reaction of a 2,6-dialkylaniline of formula II with a halide of formula III is carried out in toluene or xylene as solvent, in the presence of sodium carbonate as acid acceptor and at the reflux temperature of the reaction medium.

**16.** A process according to claim 1, wherein the reaction of an N-alkyl-2,6-dialkylaniline of formula IV with an acylating agent of formula V is carried out in an inert solvent.

**17.** A process according to claim 16, wherein the reaction of an N-alkyl-2,6-dialkylaniline of formula IV with an acylating agent of formula V is carried out in hexane, benzene, toluene, xylene, chlorobenzene, methylene chloride, chloroform, carbon tetrachloride or ethylene chloride as solvent.

**18.** A process according to claim 16, wherein the reaction of an N-alkyl-2,6-dialkylaniline of formula IV with an acylating agent of formula V is carried out in toluene or xylene as solvent.

**19.** A process according to claim 1, wherein the reaction of an N-alkyl-2,6-dialkylaniline of formula IV with an acylating agent of formula V is carried out in the absence of a base, in toluene or xylene as solvent and under reduced pressure.

**20.** A process according to claim 1, wherein the chlorination of an N-acyl-N-alkyl-2,6-dialkylaniline of formula VI is carried out in an inert solvent.

**21.** A process according to claim 20, wherein the inert solvent is formic acid, acetic acid, chlorobenzene,

methylene chloride, chloroform, carbon tetrachloride or ethylene chloride.

**22.** A process according to claim 20, wherein the inert solvent is formic acid or acetic acid with a water content of up to 40 % by weight.

**23.** A process according to claim 1, wherein the chlorination of an N-acyl-N-alkyl-2,6-dialkylaniline of formula VI is carried out in the temperature range from 20° to 40°C.

**24.** A process according to claim 1, wherein the chlorination of an N-acyl-N-alkyl-2,6-dialkylaniline of formula VI is carried out in the presence of 1 to 5 % by weight of a Lewis acid.

**25.** A process according to claim 24, wherein the Lewis acid is aluminium chloride, iron(III) chloride, boron trifluoride or titanium tetrachloride.

**26.** A process according to claim 24, wherein the Lewis acid is iron(III) chloride.

**27.** A process according to claim 1, wherein the chlorination of an N-acyl-N-alkyl-2,6-dialkylaniline of formula VI is carried out in the temperature range from 20° to 40°C, in formic acid with a water content of up to 40 % by weight and in the presence of 1.5 to 2.5 % by weight of iron(III) chloride, based on the N-acyl-N-alkyl-2,6-dialkylaniline of formula VI employed.

## Revendications

**1.** Procédé de préparation de N-acyl-N-alkyl-2,6-dialkyl-3-chloroanilinede formule I

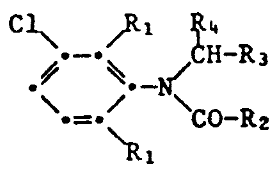

(I)

dans laquelle $R_1$ est le méthyle ou l'éthyle, $R_2$ représente un alcoxyméthyle, chlorométhyle ou 2-tétrahydrofuryle, $R_3$ un alcoxyméthyle, carboxy ou alcoxycarbonyle et $R_4$ est l'hydrogène, le méthyle et, quand $R_3$ est un carboxy ou alcoxycarbonyle, $R_4$ est aussi un 2-alcoxyéthyle et 2-alcoxypropyle, $R_3$ et $R_4$ avec l'atome de carbone auquel sont liés les deux restes, ils peuvent former aussi un reste 2-oxo-tétrahydro-3-furyle ou un reste 2-oxo-5-méthyltétrahydro-3-furyle, caractérisé en ce qu'on fait réagir une 2,6-dialkylaniline de formule II

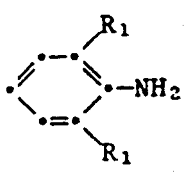

(II)

dans laquelle $R_1$ a la signification donnée pour la formule I, avec un halogénure de formule III

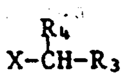

(III)

dans laquelle $R_3$ et $R_4$ ont la signification donnée pour la formule I et X est le brome ou le chlore pour donner une N-alkyl-2,6-dialkylaniline de formule IV

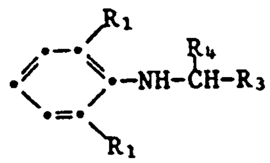

(IV)

dans laquelle $R_1$, $R_2$ et $R_4$ ont la signification donnée pour la formule I, on fait réagir ensuite la N-alkyl-2,6-dialkylaniline de formule IV avec un agent d'acylation de formule V

$X_1$ - CO - $R_2$ (V)

dans lequel $R_2$ a la signification donnée pour la formule I et $X_1$ représente le chlore, le brome ou -O-CO-$R_2$ et on transforme ensuite la N-acyl-N-alkyl-2,6-dialkylaniline de formule VI

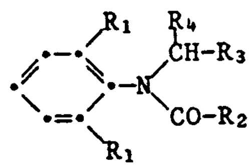

(VI)

dans laquelle $R_1$, $R_2$, $R_3$ et $R_4$ ont la signification donnée pour la formule I, par action du chlore en une N-acyl-N-alkyl-2,6-dialkyl-3-chloroaniline de formule I.

**2.** Procédé selon la revendication 1 caractérisé en ce qu'on utilise comme 2,6-dialkylaniline de formule II la 2,6-diméthylaniline ou la 2,6-diéthylaniline.

**3.** Procédé selon la revendication 2, caractérisé en ce qu'on utilise comme 2,6-dialkylaniline de formule II la 2,6-diméthylaniline.

**4.** Procédé selon la revendication 1, caractérisé en ce qu'on utilise comme halogénure de formule III le chlorure de 2-méthoxyéthyle, le chlorure de 2-éthoxyéthyle, le chlorure de 2-méthoxy-1-méthyléthyle, le 2-chloro-acétate de méthyle, le 2-chloroacétate d'éthyle, le 2-bromopropionate de méthyle ou l'$\alpha$-bromo-$\gamma$-butyrolactone.

**5.** Procédé selon la revendication 4, caractérisé en ce qu'on utilise comme halogénure de formule III l'$\alpha$-bromo-$\gamma$-butyrolactone.

**6.** Procédé selon la revendication 1, caractérisé en ce qu'on utilise comme agent d'acylation de formule V le chlorure de méthoxyacétyle, le chlorure de chloroacétyle ou le chlorure d'acide tétrahydrofuranne-2-carboxylique.

**7.** Procédé selon la revendication 6, caractérisé en ce qu'on utilise comme agent d'acylation de formule V du chlorure de méthoxyacétyle.

**8.** Procédé selon la revendication 1, caractérisé en ce qu'on réalise la réaction d'une 2,6-dialkylaniline de formule II avec un halogénure de formule III dans un solvant inerte en présence d'un agent fixateur d'acide.

**9.** Procédé selon la revendication 8, caractérisé en ce qu'on utilise comme solvant inerte du benzène, toluène, xylène, chlorobenzène, o-chlorobenzène, N,N-diméthylformamide ou N,N-diméthylacétamide.

**10.** Procédé selon la revendication 8, caractérisé en ce qu'on utilise comme solvant inerte du toluène ou du xylène.

**11.** Procédé selon la revendication 8, caractérisé en ce qu'on utilise comme agent fixateur d'acide des

hydroxydes, carbonates et hydrogénocarbonates de métal alcalin et alcalinoterreux, la pyridine ou la triéthylamine.

**12.** Procédé selon la revendication 8, caractérisé en ce qu'on utilise comme agent fixateur d'acide le carbonate de sodium.

**13.** Procédé selon la revendication 1, caractérisé en ce qu'on réalise la réaction d'une 2,6-dialkylaniline de formule II avec un halogénure de formule III à une température comprise entre 80 ° C et la température de reflux du milieu réactionnel.

**14.** Procédé selon la revendication 13 caractérisé en ce qu'on réalise la réaction d'une 2,6-dialkylaniline de formule II avec un halogénure de formule III à température de reflux du milieu réactionnel.

**15.** Procédé selon la revendication 1, caractérisé en ce qu'on réalise la réaction d'une 2,6-dialkylaniline de formule II avec un halogénure de formule III dans le toluène et le xylène comme solvant en présence de carbonate de sodium comme agent fixateur d'acide à température de reflux du milieu réactionnel.

**16.** Procédé selon la revendication 1, caractérisé en ce qu'on réalise la réaction d'une N-alkyl-2,6-dialkylaniline de formule IV avec un agent acylant de formule V dans un solvant inerte.

**17.** Procédé selon la revendication 16, caractérisé en ce qu'on réalise la réaction d'une N-alkyl-2,6-dialkylaniline de formule IV avec un agent acylant de formule V dans l'hexane, le benzène, le toluène, le xylène, le chlorobenzène, le chlorure de méthylène, le chloroforme, le tétrachlorure de carbone ou le chlorure d'éthylène comme solvant.

**18.** Procédé selon la revendication 16, caractérisé en ce qu'on réalise la réaction d'une N-alkyl-2,6-dialkylaniline de formule IV avec un agent acylant de formule V dans le toluène ou le xylène comme solvant.

**19.** Procédé selon la revendication 1, caractérisé en ce qu'on réalise la réaction d'une N-alkyl-2,6-dialkylaniline de formule IV avec un agent acylant de formule V en absence d'une base dans le toluène ou le xylène comme solvant sous pression réduite.

**20.** Procédé selon la revendication 1, caractérisé en ce qu'on réalise la chloration d'une N-acyl-N-alkyl-2,6-dialkylaniline de formule VI dans un solvant inerte.

**21.** Procédé selon la revendication 20, caractérisé en ce qu'on utilise comme solvant inerte l'acide formique, l'acide acétique, le chlorobenzène, le chlorure de méthylène, le chloroforme, le tétrachlorure de carbone ou le chlorure d'éthylène.

**22.** Procédé selon la revendication 20, caractérisé en ce qu'on utilise comme solvant inerte l'acide formique ou l'acide acétique avec une teneur en eau pouvant atteindre 40 %.

**23.** Procédé selon la revendication 1, caractérisé en ce qu'on réalise la chloration d'une N-acyl-N-alkyl-2,6-dialkylaniline de formule VI à des températures comprises entre 20 et 40 ° C.

**24.** Procédé selon la revendication 1, caractérisé en ce qu'on réalise la chloration d'une N-acyl-N-alkyl-2,6-dialkylaniline de formule VI en présence de 1-5 % pds d'un acide de Lewis.

**25.** Procédé selon la revendication 24, caractérisé en ce qu'on utilise comme acide de Lewis le chlorure d'aluminium, le chlorure de fer (III), le trifluorure de bore ou le tétrachlorure de titane.

**26.** Procédé selon la revendication 24, caractérisé en ce qu'on utilise comme acide de Lewis le chlorure de fer (III).

**27.** Procédé selon la revendication 1, caractérisé en ce qu'on réalise la chloration d'une N-acyl-N-alkyl-2,6-dialkylaniline de formule VI à 20-40 ° C dans l'acide formique ayant une teneur en eau jusqu'à 40 % en poids en présence de 1,5-2,5 % pds de chlorure de fer (III) par rapport à la N-acyl-N-alkyl-2,6-dialkylaniline de formule VI.